# EUROPEAN PATENT SPECIFICATION

(11) **EP 1 871 889 B1**
(45) Date of publication and mention of the grant of the patent: **17.10.2012**
(21) Application number: 06728396.0
(22) Date of filing: 24.02.2006
(51) Int. Cl.: A61K 9/127, C12N 7/04, C12N 15/87

(54) **A PROCESS FOR PRODUCING MODIFIED RECONSTITUTED SENDAI VIRAL ENVELOPE SPECIFIC FOR DRUG AND/OR GENE DELIVERY TO LIVER CELLS**
VERFAHREN ZUR HERSTELLUNG EINER MODIFIZIERTEN REKONSTITUIERTEN SENDAIVIRUSHÜLLE MIT SPEZIFITÄT FÜR DIE ZUFÜHRUNG VON ARZNEISTOFFEN UND/ODER GENEN AN LEBERZELLEN
PROCÉDÉ SERVANT À PRODUIRE UNE ENVELOPPE DU VIRUS SENDAI RECONSTITUÉE MODIFIÉE SPÉCIFIQUE POUR L'ADMINISTRATION DE MÉDICAMENTS ET/OU DE GÈNES À DES CELLULES HÉPATIQUES

(30) Priority: 21.04.2005 IN DE10032005
(43) Date of publication of application: 02.01.2008
(73) Proprietor: NATIONAL RESEARCH DEVELOPMENT CORPORATION, New Delhi 110 048 (IN)
(72) Inventor: VERMA, Santosh K., Gorakhpur 273 003, Uttar Pradesh (IN); MANI, Prashant, New Delhi 110 092 (IN); SHARMA, Nishi Raj, New Delhi 110 082 (IN); KRISHNAN, Anuja, New Delhi 110 091 (IN); KUMAR, Valluripalli Vinod, Safilguda, Hyderabad 500 047 (IN); REDDY, Bathula, Surendar, Nalgonda 508 211, Andhra Pradesh (IN); CHAUDHURI, Arabinda, Hyderabad 500 076 (IN); ROY, Rajendra P., Aruna ASAF Ali Marg, New Delhi 110 067 (IN); SARKAR, Debi P., Type VB, Flat , 6A Ground Floor, New Delhi 110 021 (IN)
(74) Representative: Green, Mark Charles
(86) International application number: PCT/IN2006/000061
(87) International publication number: WO 2006/111982

(56) References cited:
- JP-A- 2002 065 278
- US-A- 5 683 866
- VERMA SANTOSH K ET AL: "Histidylated lipid-modified Sendai viral envelopes mediate enhanced membrane fusion and potentiate targeted gene delivery" JOURNAL OF BIOLOGICAL CHEMISTRY, vol. 280, no. 42, October 2005 (2005-10), pages 35399-35409, XP002592939 ISSN: 0021-9258
- CHO J E ET AL: "Enhanced cytotoxicity of doxorubicin encapsulated in liposomes with reconstituted Sendai F-proteins" JOURNAL OF MICROENCAPSULATION, vol. 18, no. 4, July 2001 (2001-07), pages 421-431, XP002592940 ISSN: 0265-2048
- BAGAI SANGEETA ET AL: "Hemagglutinin-neuraminidase enhances F protein-mediated membrane fusion of reconstituted Sendai virus envelopes with cells" JOURNAL OF VIROLOGY, vol. 67, no. 6, 1993, pages 3312-3318, XP002592941 ISSN: 0022-538X
- KUMAR V.V. ET AL. GENE THER. vol. 10, no. 15, August 2003, pages 1206 - 1215, XP003002109
- KIM H.S. ET AL. CANCER GENE THER. vol. 9, no. 2, February 2002, pages 173 - 177, XP003002110

## Description

### FIELD OF INTENTION

This invention relates to a process for producing modified reconstituted Sendai viral envelope specific for drug and/or gene delivery to liver cells.

### PRIOR ART

Completion and annotation of the human genome sequence has significantly enhanced the possibility of using designer DNA for gene therapy. Nonetheless, in spite of first gene therapy trial fourteen years ago, and numerous clinical trials thereafter, successful gene therapy remains elusive. The major problem towards ensuring clinical success of gene therapy is to design safe, efficacious and target-specific transfection vectors. Towards designing such gene carriers, significant progress has been made on gene transfection by novel non-glycerol-based histidylated cationic amphiphiles, presumably via the endosome-disrupting properties of the histidine functionalities. Histidine residues in amphipathic peptide antibiotics are also known to promote efficient DNA delivery into mammalian cells in vitro by favoring endosomal escape of the DNA molecules at low pH. Complexes of recombinant adenoviruses and cationic lipids/polymers have been tested for the enhancement of therapeutic index for the treatment of cystic fibrosis. The beneficial role of such complexes was clearly envisaged by the amplification of binding and entry of adenovirus particles into host cells. However, despite all these encouraging results, its failure in targeted gene transfer in whole animal and inherent cytopathic/cytotoxic side effects limit its applications in liver gene therapy.
To obviate such a disadvantage, U.S. patent specification no. 5683866 teaches a process for producing a reconstituted Sendai viral envelope containing the F-protein. Such a reconstituted envelope has the specific application of a targeted delivery and expression of a foreign reporter gene into liver cells.

The process for producing the reconstituted Sendai viral envelope and as described in the aforesaid patent consisted in adding a reducing agent to Sendai virus to reduce the HN protein and to produce a reduced virus. The reduced virus is subjected to the step of dialysis for removal of the reducing agent. The reduced virus is solubilized to obtain a solution and from which the insolubles containing reduced HN protein and the core are removed. The required nucleic and gene or drugs is added to the supernatant and the soulubilizing agent is then removed therefrom. Such a reconstituted Sendai viral envelope had the distinct advantage of it being specific as a target delivery system for a drug or gene specific to liver cells. However, it has now been found that the efficiency of the known reconstituted Sendai viral envelope needs to be increased. One of the reasons that may be attributed for the comparatively lower efficiency of the known reconstituted Sendai viral envelope is that the own gene of the virus, which has the property of replication, is removed so as to allow the new gene to be introduced into the envelope.
It is believed that such a removal of the gene along with its HN protein may be one of the reasons attributing to the comparative lowering of the efficiency.

### OBJECTS OF THE INVENTION

An object of this invention is to propose a process for producing modified reconstituted Sendai viral envelope specific for drug and/or gene delivery to liver cells.

Another object of this invention is to propose a process for producing modified reconstituted Sendai viral envelope which is more efficient than the known Sendai viral envelopes.

Still another object of this invention is to propose a delivery system specific to liver cells which is non-pathogenic to humans.

### BRIEF DESCRIPTION OF INVENTION

According to this invention there is provided a process for producing a targeted gene and/or drug delivery system for liver cells comprising the steps of:
i. chemical reduction of Sendai virus for reduction of HN protein;
ii. subjecting the reduced virus to the step of dialysis for removal of the reducing agent;
iii. solubilizing the reduced virus with a detergent to obtain a solution;
iv. centrifuging the solution to separate the insolubles consisting of reduced HN protein and core of said virus;
v. adding histidylated lipid to the supernatant;
vi. adding the drug or gene after addition of the lipid;
vii. removing the detergent from the envelope and then subjecting it to the step of centrifugation to obtain His lipid F-virosomes with entrapped drug or DNA.

Further, according to this invention there is provided a process for the delivery of DNA and other biological macromolecules into HepG2 cells using F-virosomes comprising in the steps of:
i. growing sendai virus (Z-strain);
ii. harvesting and purifying the Sendai virus by any known method;
iii. preparing reconsistituted Sendai viral envelopes containing the F-protein (F-virosomes);
iv. incorporating Histidylated lipid into the reconstituted Sendai virus envelope containing at this stage only the solubles including F-protein;
v. adding the required gene or drug;
vi. removing the detergent and then subjecting it to the step of centrifugation.

Specifically, the process of the present invention resides in the step of incorporating the envelope with Histidylated lipid, and, only after removal of the gene of Sendai virus alongwith insolubles including HN protein. It has been found that to obtain an efficacy, an incorporation of the envelope with Histidylated lipid should be effected after removal of the gene of Sendai virus alongwith insolubles including HN protein. Firstly, Histidylated lipid is not soluble in water, and if introduced in the very initial stage of the process, the lipid would not be retained in the supernatant. Secondly only a removal of the Sendai virus alongwith insolubles including HN protein would allow an introduction of Hitidylated lipid. Thirdly replacing the HN protein with HIS-lipid in increases the efficiency of the existing gene courier without affecting the liver specificity.

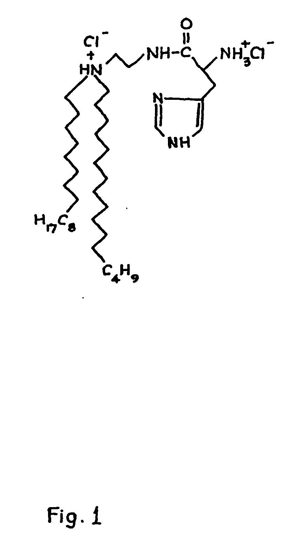

Without intending to imply any limitation, Histidylated lipid has a chemical formula as shown in Fig. 1 of the accompanying drawings (L-histidine (N,N,-di-n-hexadecylomine) ethylamide.

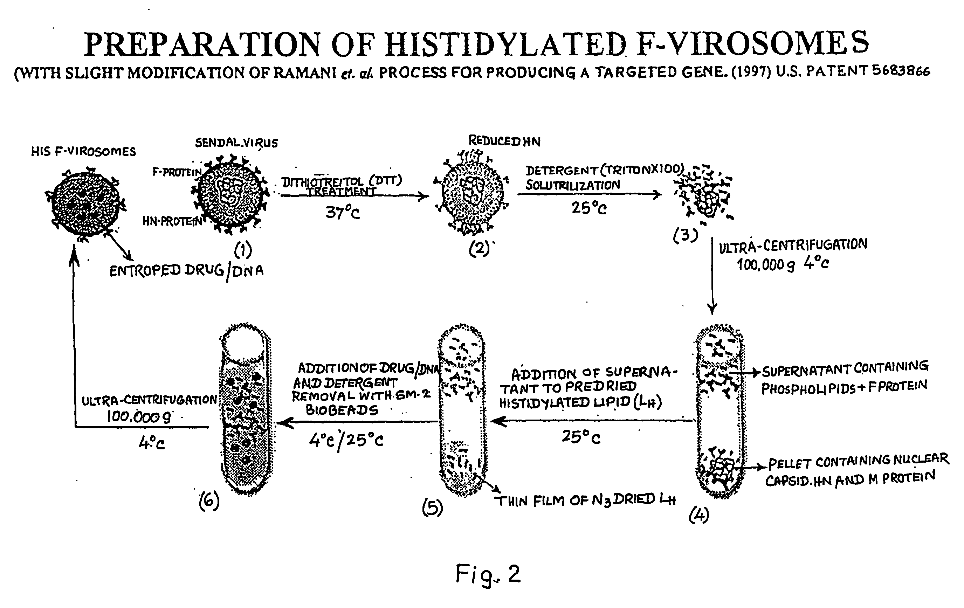

Fig. 2 of the accompanying drawing show the flow diagram of the prowess. Steps (1) to (4) are known from the afore said patent. As will be apparent, step (4) involves the removal of the insolubles containing the Sindus virus nuclear capsid, HN and M protein. The supernatant obtained from such a step of removal contains the phospholipids and F protein. Specifically, and referring to step (5) Histidylated lipid is introduced followed by step (6) of addition of drug/DNA and removal of detergent.

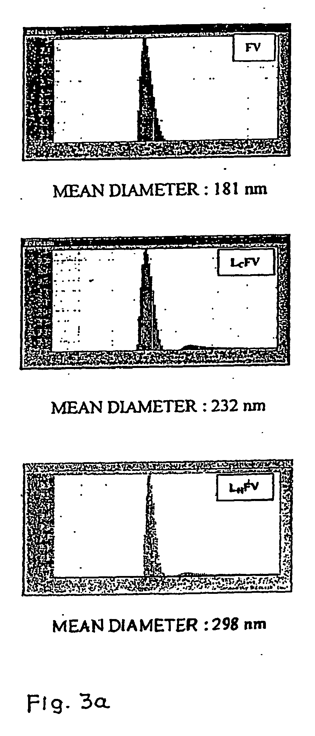

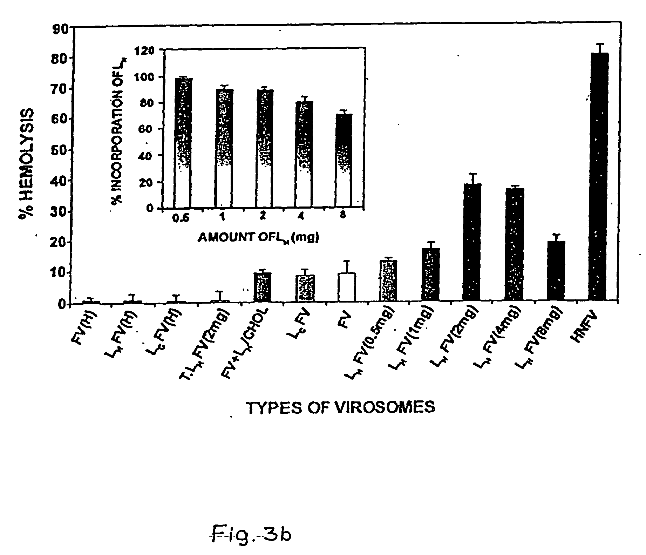

In order to evaluate the particle sizes, the virosomal preparations were subjected to photon correlation spectrometry (PCS) measurements. It revealed the mean diameter of the FVs to be 181 nm, which conforms well to the average size reported earlier by transmission electron microscopy, on the other hand, L_{H}FV and L_{C}FV preparations were found to be of 298 nm and 232 nm respectively (Fig. 3a). The membrane fusion activity of F protein in FV is well correlated with its ability to hemolyse various RBCs . As shown in Fig. 3b, various doses of L_{H} molecules significantly enhanced the hemolytic activity of L_{H}FVs. With 2 mg of L_{H} incorporated in to 50 mg of total Sendai virus protein resulted in four fold increase in hemolytic activity. The specificity of the effect of L_{H} was established by parallel control with L_{C} (having the same amount of positively charged head group but lacks histidine moiety, Fig. 1) which did not affect hemolysis over FVs alone. Strikingly, same amount of L_{H} in L_{H}/CHOL liposomes mixed with FVs did not affect hemolysis over FVs alone. HNFVs, containing HN that markedly enhances its lytic activity served as positive control and incorporation of L_{H} and L_{C} molecules in HNFVs did not enhance the hemolytic activity any further. Similar results were also obtained with RBCs from human and rabbit (data not shown). The HPLC analysis of the sucrose density gradient purified virosomes showed 85% efficiency of incorporation using 2 mg of L_{H} against 50 mg of Sendai virus (Fig. 3b). The gradient purification of virosomes ensured the co-grafting of L_{H} and L_{C} molecules with F protein in the viral membrane. Similar profile of incorporation (and size) was also obtained with HAV and GV (data not shown). From Ni-affinity purification profile followed by protein assay, more than 98% of the L_{H}FV particles were found to retain L_{H} molecules. The incorporation profile of L_{C} in FVs followed the same trend (data not shown). Calculation based on the protein/lipid composition of virosomes, molecular weights of F (HA and G) and that of the cationic lipids, about 60 molecules L_{H} (and L_{C}) were estimated to be associated with one molecule of F (HA and G) protein in the respective virosomal membranes (having 2 mg of L_{H} with 50 mg of Sendai, Influenza and VSV). All further experiments were performed at this stochiometry.

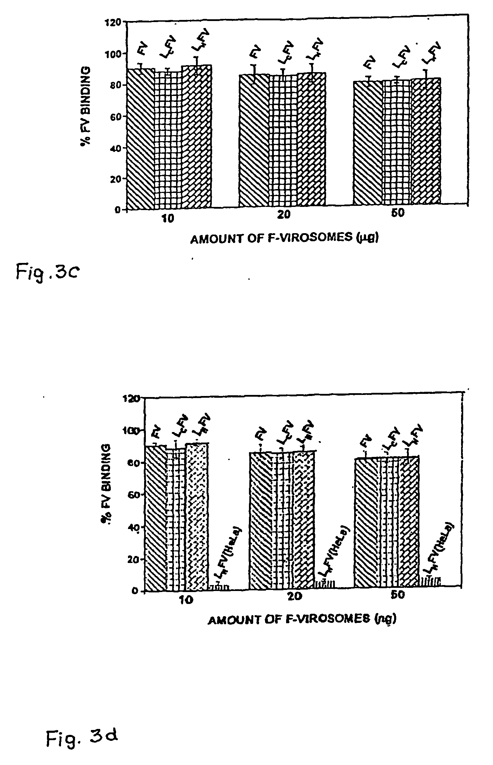

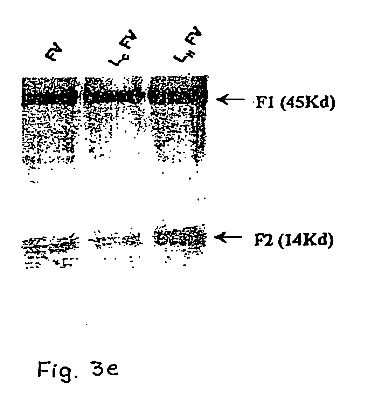

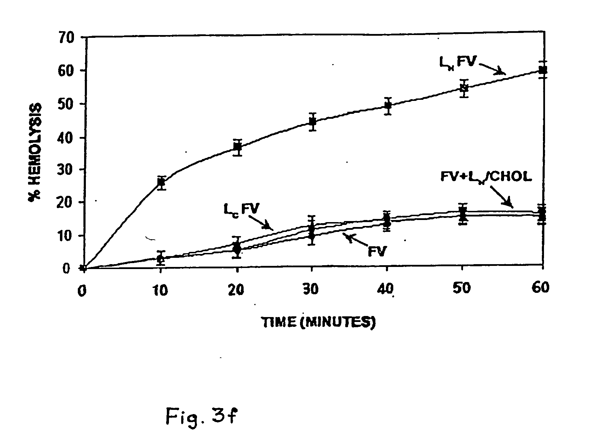

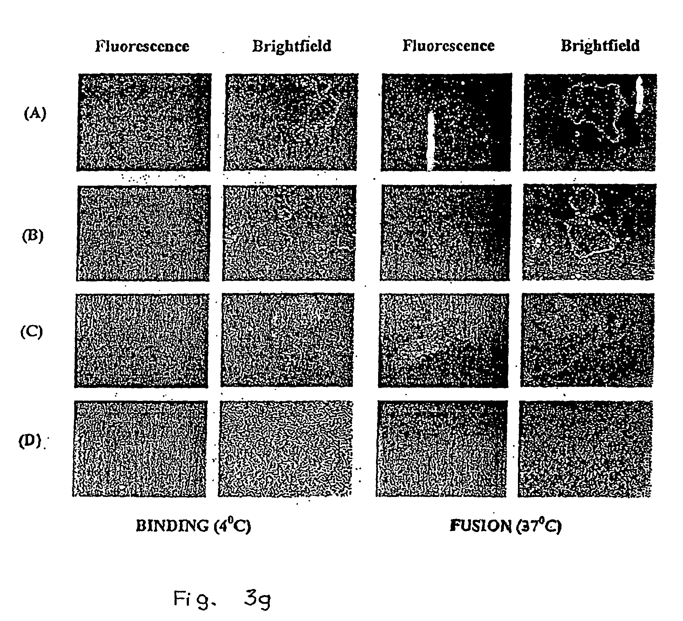

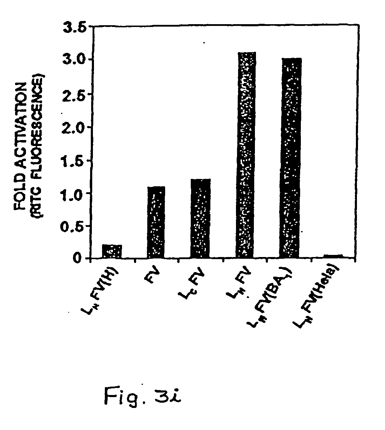

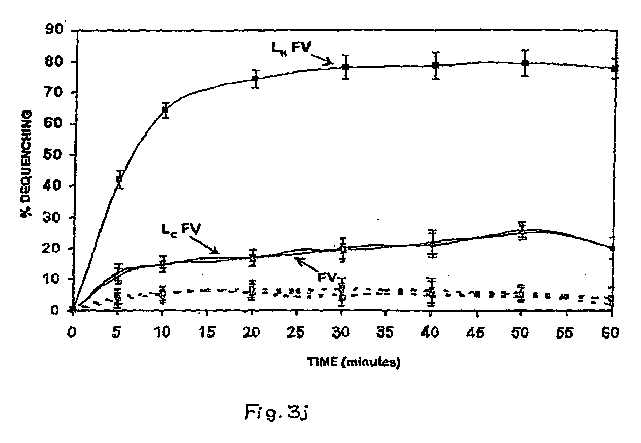

NBD-PE-labeled FVs, L_{H}FVs and L_{C}FVs, contained equivalent ampount of F protein of identical electrophoretic mobility (Fig. 3e), had equal affinity for the mouse RBCs and HepG2 cells but showed negligible attachments to HeLa cells at 37°C (Fig. 3c and 3d). As shown in Fig. 3f, presence of L_{H} in the viral membrane markedly enhanced the kinetics (~ eight fold increase) as well as the extent of hemolysis. L_{H}/HOL liposomes mixed with FVs failed to exert such effect. Fluorescent micrographs showing escalated movement of NBD-PE (dequenching of fluorescence) from L_{H}FVs to mouse RBCs indicated membrane fusion (compare panel A, B with C in Fig. 3g) while the transfer of RITC-lysozyme from loaded-virosomes to an increased number of HepG2 cells (compare panel 1, 2 with 3 in Fig. 3i) indicated core mixing. This also corroborated the fast kinetics (almost four fold higher rate) and higher extents of fluorescence dequenching of NBD-PE (Fig. 3j) with L_{H}FV/target cells at neutral pH. Such movement of NBD-PE was not observed with FVs mixed with L_{H}/CHOL liposomes (data not shown). It was also confirmed that at low temperature (4°C), L_{H} in virosomal membrane did not have any influence on binding to RBCs (Fig. 3g, panels A, B, C) and HeLa cells (Fig. 3d). Finally, experiments carried out with DNA loaded-virosomes at pH 5.0 showed similar results (data not shown).

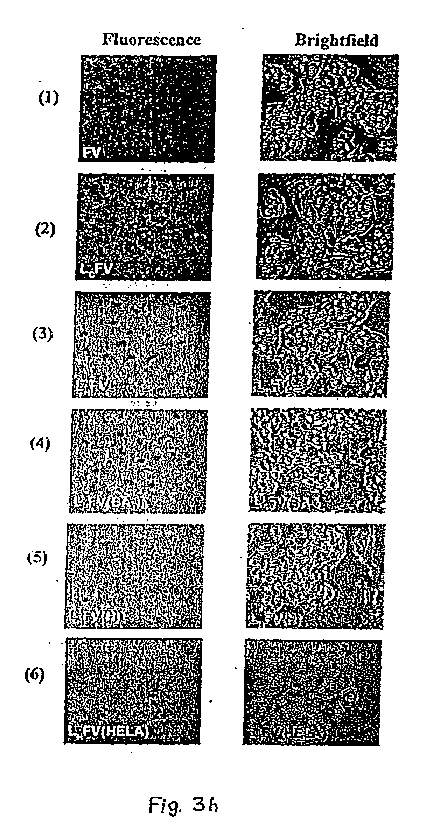

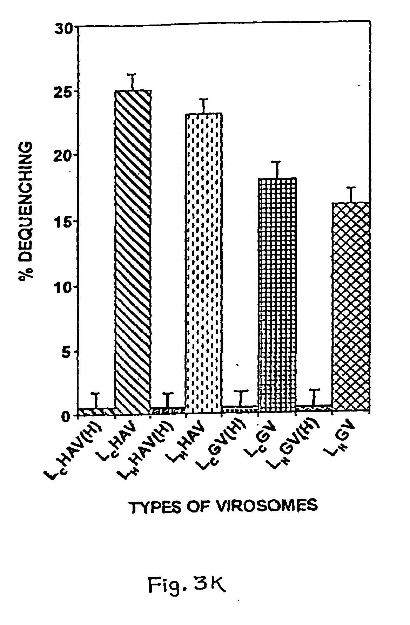

Bafilomycin A₁, that inhibits endosome acidification by blocking the action of H⁺-ATPase, could not diminish the fusion-mediated RITC-lysozyme transfer from L_{H}FVs (Fig. 3h, panel#4, during co-incubation, on the other hand, 15 min pre- and post-incubation condition results same as that of co-incubation, data not shown). L_{H}/CHOL liposomes, when co-incubated with all types of loaded-virosome/target cell complexes, did not affect at all the F-protein-mediated membrane fusion (membrane and core mixing events) at both pH 7.4 and 5.0 (data not shown). Notably, L_{H} could not influence the activation of HA and G protein induced fusion of viral envelopes with HepG2 cells (Fig. 3k).

### L_{H} CONTAINING F-VIROSOMES AUGMENT TARGETED GENE TRANSFER IN VITRO.

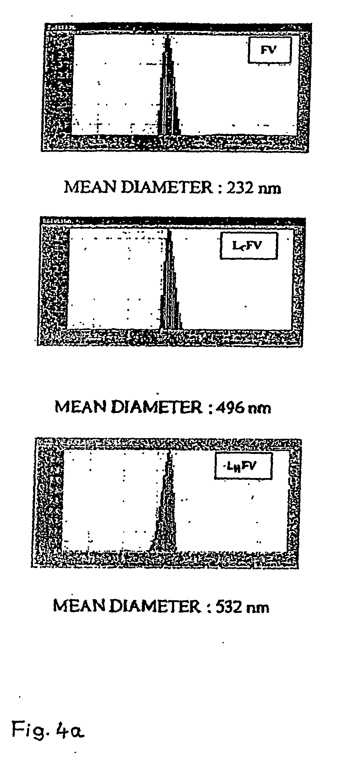

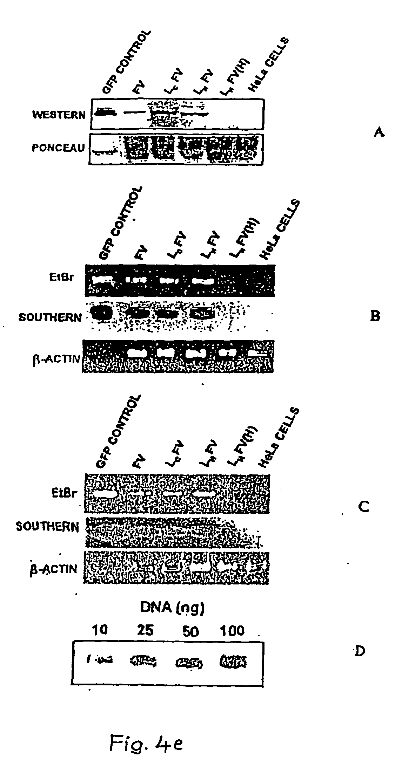

To assess the effect of DNA loading on the size of various virosomes, the preparations were checked by PCS-analysis. Upon entrapment of pEGFP-N1 DNA in FVs (50µg DNA/ mg of F protein), the size of the loaded-virosomes showed a slight increase to 232 nm, whereas, same amount of DNA loaded in L_{C}FV_{S} and L_{H}FVs (80µg DNA/ mg of F protein) exhibited almost two-fold increase (Fig. 4a). However, as observed with FVs earlier, all three types of FV associated DNA was found to be DNase I resistant, indicating that it was entrapped rather than adsorbed on the virosomal membrane. No detectable leakage of DNA was observed from such loaded vesicles incubated with PBS or mouse plasma/FCS at 37°C for about 6 h and heat-treated virosomes (56°C for 30 min). Membrane fusion activity of these loaded preparations was assessed by their ability to lyse mouse RBCs and fuse with HepG2 cells in culture and found to be very similar to that of, as described above, their unloaded counterparts (data not shown).

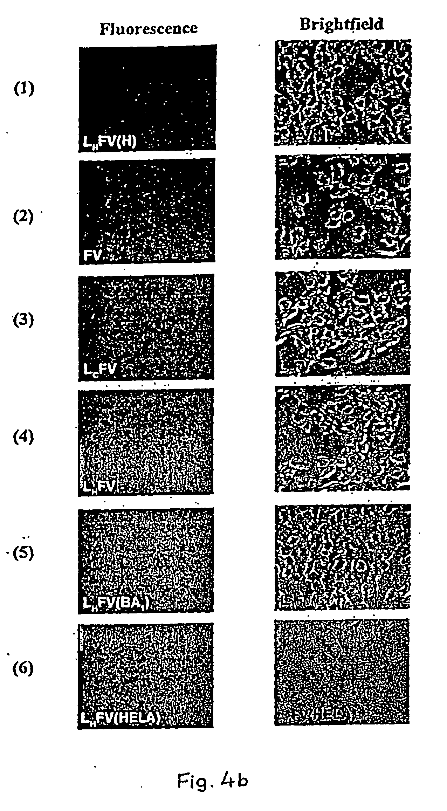

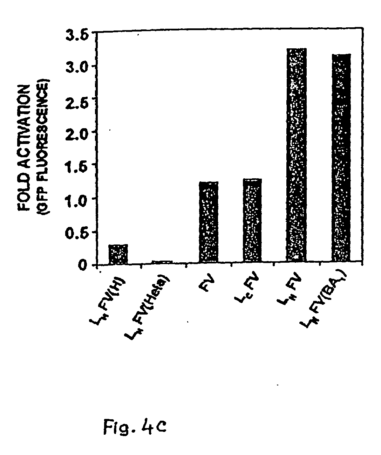

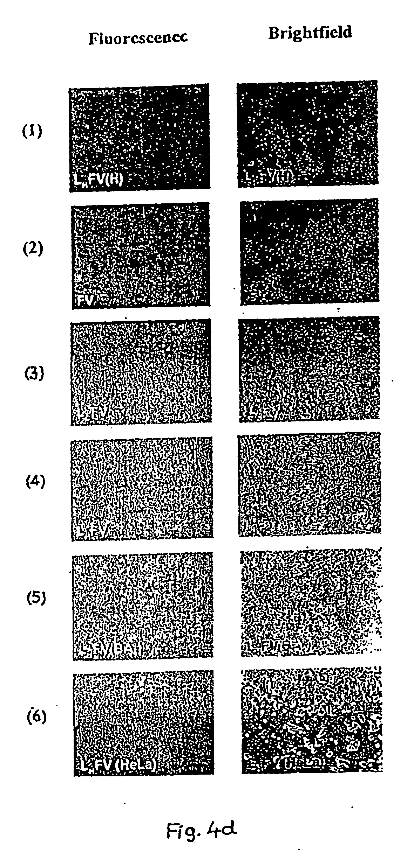

With a view to evaluate the efficiency of gene expression mediated by L_{H}FVs, 4 µg of pEGFP-N1 DNA-loaded virosomes were incubated with HepG2 and HeLa cells for 24 h in the culture medium. Almost 60-80 % of the HepG2 cells expressed EGFP protein as shown in Fig. 4b, panel #4 under the influence of L_{H} lipid, whereas FV and L_{C}FVs could exert an equal efficiency of 20-30 % EGFP positive cells (panel# 2 and 3 respectively). Three to four fold enhancement of gene expression is corroborated by quantitating fluorescence in the cell extracts (Fig. 4c). Heat-treated L_{H}FVs (panel#1) and L_{H}FVs incubated with HeLa cells (panel#6) failed to express EGFP. However, in the presence of bafilomycin A₁, the transfection efficiency remained unchanged (panel#5) as observed in case of lysozyme transfer (Fig. 3h). These results were further verified with immunofluorescence studies (Fig. 4d) with specific anti-GFP antibody upon fixing the same cells as in Fig. 4b. To obtain further support of expression profile of EGFP expression in various treatments, the cells were processed for western blot analysis (Fig. 4e, panel A) and a high level expression (ca. 3 fold more) was reconfirmed in case of loaded L_{H}FV_{S}. In order to determine whether the observed differences in gene expression were due to varying efficiencies of DNA delivery and differential transcript synthesis, DNA-PCR and RT-PCR were performed, followed by Southern analysis from the cells in a parallel experiment (results presented in Fig. 4e, panel B and C respectively). A careful comparision of the Southern hybridized specific band intensities with a standard curve (Fig. 4e, panel D), revealed approximately 2½ fold more delivery of EGFP DNA under L_{H} influence, keeping the number of HepG2 cells fixed. Nevertheless, a direct correlation between the amount of delivered DNA and the level of transgene expression was observed.

### SITE-SPECIFIC GENE DELIVERY IN WHOLE ANIMAL THRONGH L_{H}FV.

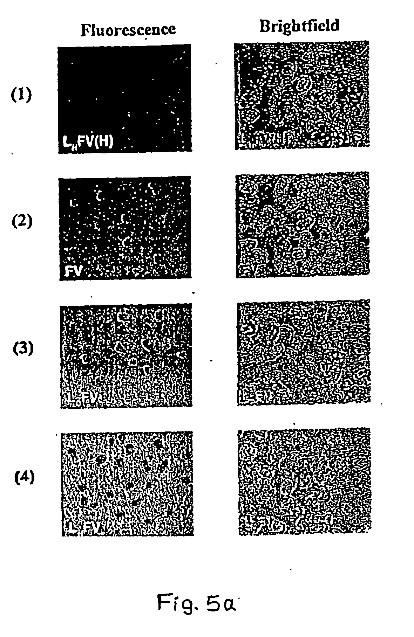

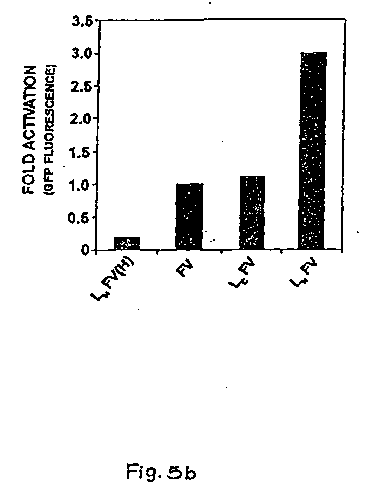

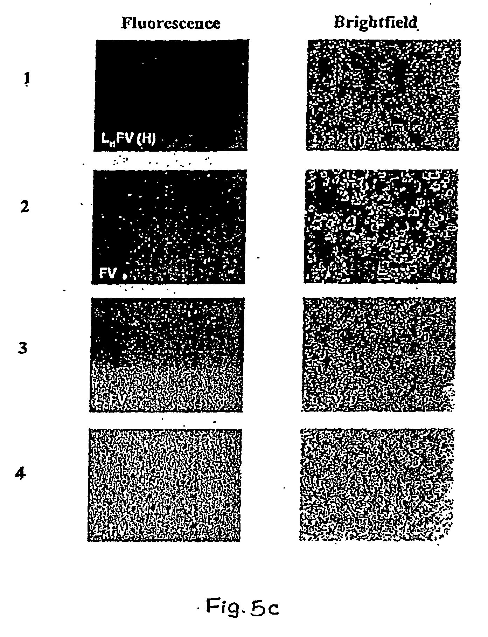

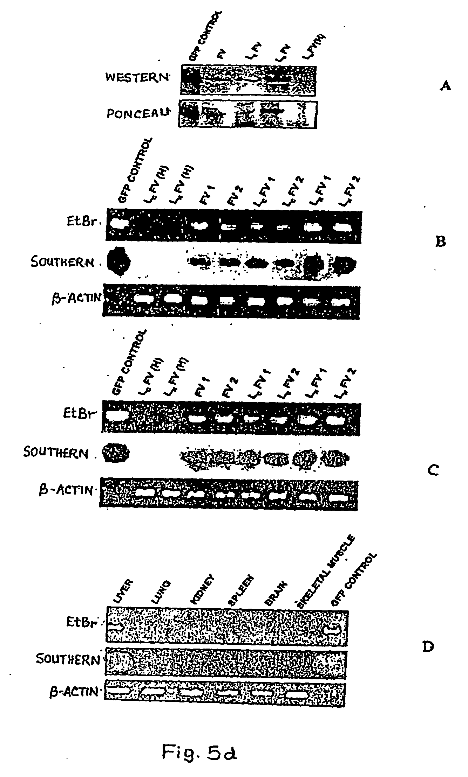

To ensure that the transgene expression was exclusive for hepatocytes (in conformity with the cell-specific nature of the virosomal delivery system, in vivo), cells isolated from various organs of mouse, 2 days after injection of 4 µg DNA loaded virosomes were analyzed for EGFP gene expression. This was crtically assessed in intact liver parenchymal cells both at the level of protein (Fig. 5a, b, c and d, panel A), DNA-PCR (4d, panel B) and RT-PCR (4d, panel C). A remarkable transfection efficiency (> 80% of the hepatocytes) was achieved with histidylated virosomes (a, panel#4) in terms of protein in liver cells as compared with that of FVs (a, panel#2) and L_{C}FVs (a, panel#3) in mice. Heat-treated L_{H}FVs failed to show detectable gene expression as expected (a, panel#1). Similar fold increase in EGFP expression was substantiated from the fluorescence quantitation (Fig. 5b). This superior nature of histidyl induction of fusion-mediated cytosolic delivery, *in vivo*, was further corroborated with immunofluorescence analysis with the fixed cells (Fig. 5c) and western analysis (ca. 2.5 fold more over controls) in parallel experiments (Fig. 5d, panel A). DNA-PCR analysis of two mice (4d, panel B) (and repeated three times) reflected almost two fold more delivery of DNA by loaded L_{H}FVs over FVs and L_{C}FVs to the hepatocytes. RT-PCR analysis of total RNA from isolated hepatocytes (4d, panel C) matched higher DNA delivery. Failure of the detection of transcripts in other organs (4d, panel D) confirmed the presence of EGFP-specific transcripts in mouse liver only. Moreover, total lymphocytes from blood and cells isolated from the organs (as in Fig. 5d, panel D) other than liver did not exhibit any EGFP fluorescence (data not shown). The animals remained healthy and active during the experiments.

### HISTIDYLATED CATIONIC LIPID INDUCES CONFORMATIONAL CHANGES IN F PROTEIN.

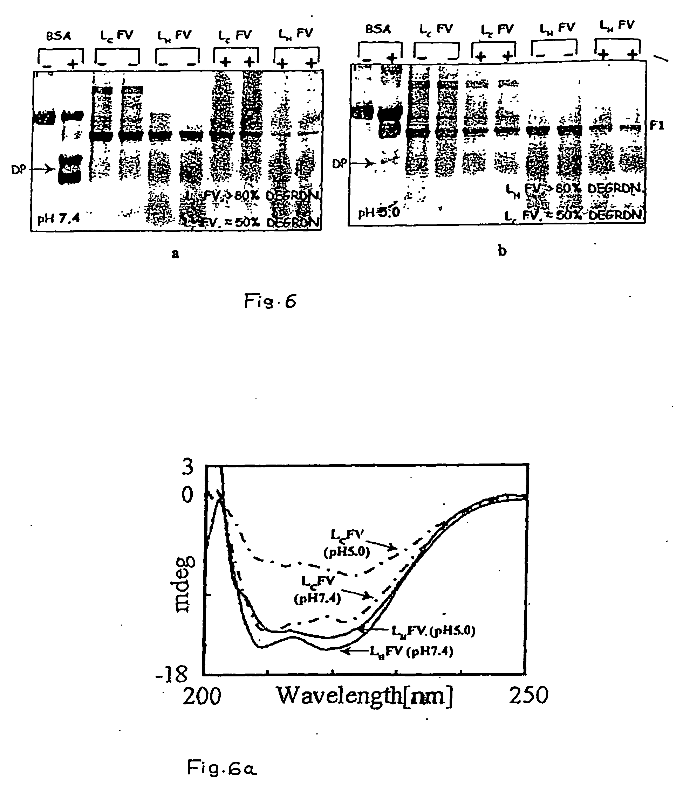

Limited proteolysis experiments have been successfully used to probe changes in structure, dynamics and function of proteins. We subjected L_{H}FV and L_{C}FV to proteinase K digestion to ascertain conformational changes in F protein due to the presence of the histidylated lipid, which might have enhanced the membrane fusogenic propensity of F protein. Accordingly, proteolysis of F protein was carried out at pH 7.4 and 5.0 respectively. The SDS-PAGE analysis of the proteinase K digest shown in Fig. 6 (a and b) reveals that proteolysis of F protein occurs in much the same way at both the pH. However, the F protein is significantly more susceptible to proteinase K digestion in the presence of histidylated lipid. Under identical conditions, about 80% of the protein is digested in L_{H}FV as compared to only 50% in L_{C}FV. These results are indicative of a conformational change in F protein in the presence of the histidylated lipid. Since L_{H}FV was relatively more susceptible to proteinase K digestion as compared with the L_{C}FV, we examined the influence of histidylated lipid on the conformation of F protein by CD spectroscopy (Fig. 6c). For this, we examined the far UV CD spectra of the F protein in the presence and in the absence of histidylated lipids. At both pH values (7.4 and 5.0), the spectra of the F protein exhibited double minima at 222 and 208 nm that are characteristics of helical structure. The ellipticity value at 222 nm for F protein in the presence of L_{H}FV at pH 7.4 was about 15% higher than that of L_{C}FV. At pH 5.0, this difference was magnified to about 50% relative to L_{C}FV. Although, slight decrease in negative ellipticity was noticeable in L_{H}FV sample at pH 5.0 as compared with pH 7.4, the dramatic diminution seen in the case of L_{C}FV at pH 5.0 indicate that histidylation indeed significantly contributed to the stabilization of the secondary structure of the F protein.

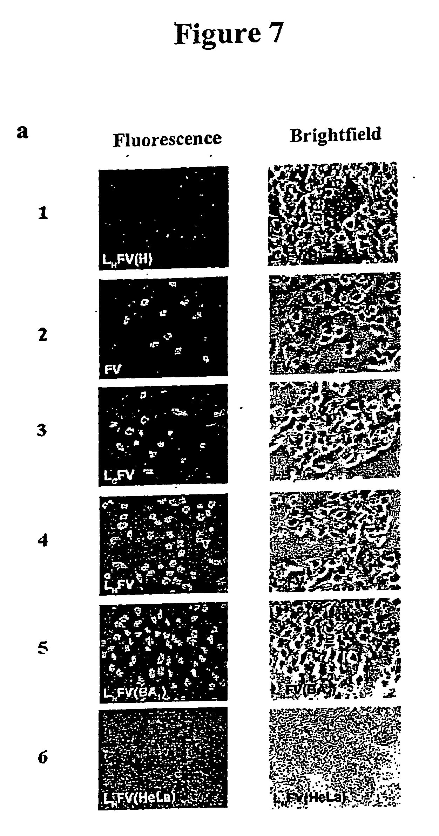

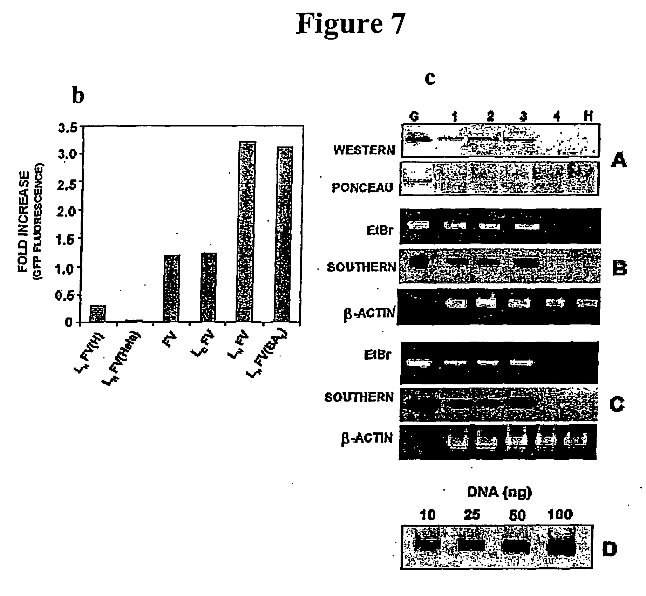

Fig. 7: shows the luciferase gene expression profiles in isolated Parenchymal cells from injected mice.

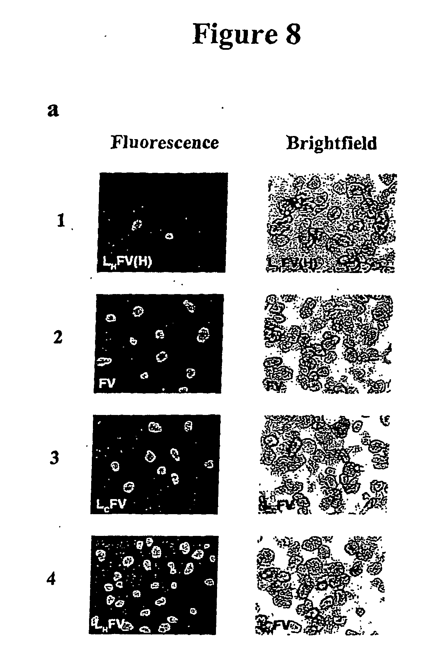

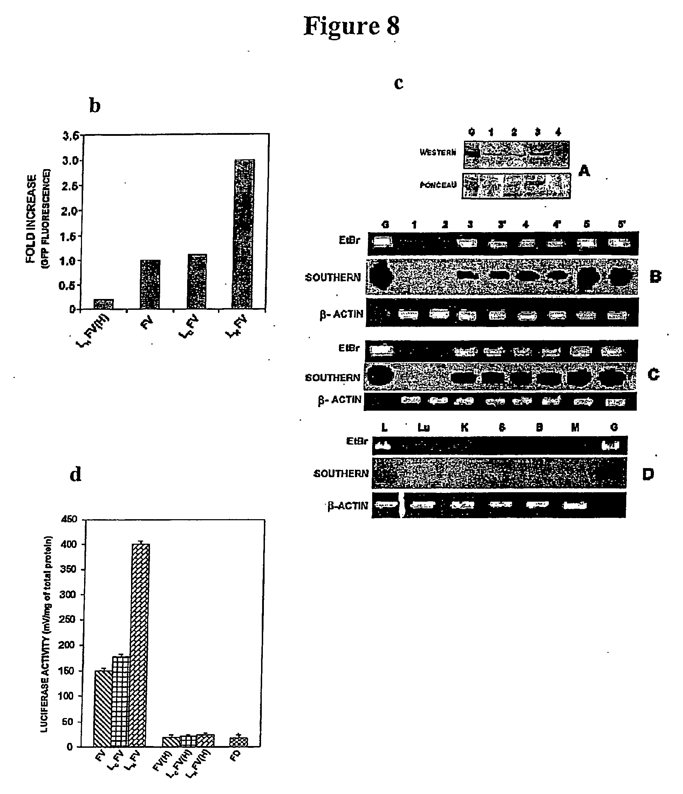

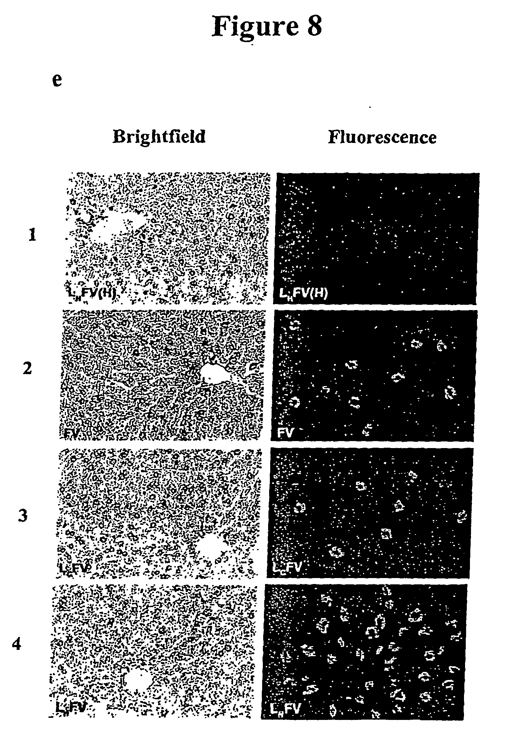

and Fig.8 :shows the immunohisto-chemical analysis of EGFP expression in liver sections.

### EXAMPLE 1

**Preparation of Histidylated Lipid-Modified loaded F-virosomes (L_{H} FV**) - F-virosomes were prepared following published/patented (**Process for Producing a Targeted Gene** (1997) U.S. Patent 5,683,866) procedures. Cationic amphiphiles (L_{H} and L_{C}) were incorporated in reconstituted viral membrane as described earlier for making NBD-PE labelled virosomes (Bagai, S., Puri, A., Blumenthal, R. and Sarkar, D.P. (1993) J. Virol. 67, 3312-3318). Briefly,the L_{H} lipid (2 to 8 mg) dissolved in solvents (chloroform: methanol = 2:1 v/v) were dried in a glass vial separately under nitrogen to form a thin film. The supernatant (2.5 to 10 ml) from the detergent extract of pure Sendai virus (50 to 200 mg protein), containing only the viral F protein (3.0 to 12 mg) and the lipids, was added to the L_{H} film and incubated at 20 °C for 30 min with gentle shaking. The detergent was removed by SM2 Bio-Beads, to form the virosomes. The amount of amphiphiles incorporated into the virosomal membranes (L_{H}FV) was analyzed by HPLC after purifying the virosomal preparations through a continuous sucrose gradient as described elsewhere. The plasmid pEGFP-N1 (Clontech, USA, isolated using Qiagen Megaprep unit) coding for enhanced green fluorescent protein (EGFP) under the control of cytomegalovirus (CMV) promoter and RITC-Lysozyme were entrapped in **L_{H} FV** following our patented protocol as above. The virosome preparations such formed, were passed through 26-gauge needle 20 times and their particle size analyzed (532 nm) by a Photon Correlation Spectrometer (PCS, Model: Photocor FC, USA) using the software Flexcor/DynaLS provided by the Photocor company. The system was calibrated using the 200 nm silica/Latex particles (supplied by Photocor FC).

## Claims

1. A process for producing a targeted gene and/or drug delivery system for liver cells comprising the steps of:
i. chemical reduction of Sendai virus for reduction of HN protein;
ii. subjecting the reduced virus to the step of dialysis for removal of the reducing agent;
iii. solubilizing the reduced virus with a detergent to obtain a solution;
iv. centrifuging the solution to separate the insolubles consisting of reduced HN protein and core of said virus;
v. adding histidylated lipid to the supernatant;
vi. adding the drug or gene after addition of the lipid;
vii.removing the detergent from the envelope and then subjecting it to the step of centrifugation to obtain His lipid F-virosomes with entrapped drug or DNA.

2. A process for the delivery of DNA and other biological macromolecules into HepG2 cells using F-virosomes comprising in the steps of:
i. growing Sendai virus (Z-strain);
ii. harvesting and purifying the Sendai virus by any known method;
iii. preparing reconstituted Sendai viral envelopes containing the F-protein (F-virosomes);
iv. incorporating Histidylated lipid into the reconstituted Sendai virus envelope containing at this stage only the solubles including F-p protein;
v. adding the required gene or drug;
vi. removing the detergent and then subjecting it to the step of centrifugation.

3. A process as claimed in claim 1 wherein the histidylated lipid is L-histidine (N,N-di-n-hexadecylamine) ethylamide.

4. A process as claimed in claim 2 wherein the histidylated lipid is L-histidine (N,N-di-n-hexadecylamine) ethylamide.

5. A process as claimed in claim 1 wherein 2mg of histidylated lipid is added to every 25 ml of supernatant containing 3 mg of viral F protein.

## Patentansprüche

1. Verfahren zur Herstellung eines Systems für gezielte Gen- und/oder Wirkstoffabgabe für Leberzellen, wobei das Verfahren die folgenden Schritte umfasst:
i. chemisches Reduzieren eines Sendaivirus zum Reduzieren von HN-Protein;
ii. Unterziehen des reduzierten Virus dem Dialyseschritt zum Entfernen des Reduktionsmittels;
iii. Solubilisieren des reduzierten Virus mit einem Detergens, um eine Lösung zu erhalten;
iv. Zentrifugieren der Lösung, um die unlöslichen Stoffe, die aus reduziertem HN-Protein und dem Kern des Virus bestehen, abzutrennen;
v. Zugeben von histidyliertem Lipid zu dem Überstand;
vi. Zugeben des Wirkstoffs oder Gens nach Zugabe des Lipids;
vii. Entfernen des Detergens aus der Membran und dann Unterziehen dieses dem Zentrifugationsschritt, um His-Lipid-F-Virosome mit eingeschlossenem Wirkstoff oder eingeschlossener DNA zu erhalten.

2. Verfahren zur Abgabe von DNA und anderen biologischen Makromolekülen in HepG2-Zellen unter Verwendung von F-Virosomen, wobei das Verfahren die folgenden Schritte umfasst:
i. Züchten eines Sendaivirus (Z-Stamm);
ii. Ernten und Aufreinigen des Sendaivirus mit einem beliebigen bekannten Verfahren;
iii. Herstellen von rekonstituierten Sendaivirusmembranen, die das F-Protein (F-Virosome) enthalten;
iv. Integrieren von histidyliertem Lipid in die rekonstituierte Sendaivirusmembran, die in dieser Stufe nur die löslichen Stoffe enthält, einschließlich F-p-Protein;
v. Zugeben des erforderlichen Gens oder Wirkstoffs;
vi. Entfernen des Detergens und dann Unterziehen dieses dem Zentrifugationsschritt.

3. Verfahren nach Anspruch 1, wobei das histidylierte Lipid L-Histidin-Ethylamid (N,N-Di-n-hexadecylaminethylamid) ist.

4. Verfahren nach Anspruch 2, wobei das histidylierte Lipid L-Histidin-Ethylamid (N,N-Di-n-hexadecylaminethylamid) ist.

5. Verfahren nach Anspruch 1, wobei 2 mg histidyliertes Lipid jeweils 2,5 ml Überstand, der 3 mg Virus-F-Proteine enthält, zugegeben werden.

## Revendications

1. Procédé pour la production d'un système d'administration ciblée de gène et/ou de médicament pour les cellules hépatiques comprenant les étapes consistant à :
i. effectuer la réduction chimique du virus Sendai pour effectuer la réduction de la protéine HN ;
ii. soumettre le virus réduit à une étape de dialyse pour enlever l'agent réducteur ;
iii. solubiliser le virus réduit avec un détergent pour obtenir une solution ;
iv. centrifuger la solution pour séparer les matières insolubles constituées de la protéine HN réduite et du coeur dudit virus ;
v. ajouter un lipide histidylé au surnageant ;
vi. ajouter le médicament ou le gène après ajout du lipide ;
vii. enlever le détergent de l'enveloppe et ensuite la soumettre à une étape de centrifugation pour obtenir des F-virosomes à His-lipide contenant un médicament ou ADN encapsulé.

2. Procédé pour l'administration d'ADN ou d'autres macromolécules biologiques dans des cellules HepG2 utilisant des F-virosomes comprenant les étapes consistant à :
i. faire croître le virus Sendai (souche Z) ;
ii. récolter et purifier le virus Sendai par n'importe quelle technique connue ;
iii. préparer des enveloppes de virus Sendai reconstituées contenant la protéine F (F-virosomes) ;
iv. incorporer un lipide histidylé dans l'enveloppe de virus Sendai reconstituée ne contenant à ce stade que les matières solubles dont la protéine F-p ;
v. ajouter le gène ou médicament requis ;
vi. enlever le détergent et ensuite la soumettre à une étape de centrifugation.

3. Procédé selon la revendication 1 dans lequel le lipide histidylé est le *(N,N-di-n-*hexadécylamine)éthylamide de la L-histidine.

4. Procédé selon la revendication 2 dans lequel le lipide histidylé est le (*N,N-di-n-*hexadécylamine)éthylamide de la L-histidine.

5. Procédé selon la revendication 1 dans lequel 2 mg de lipide histidylé sont ajoutés pour 2,5 ml de surnageant contenant 3 mg de protéines F virales.
